# EUROPEAN PATENT APPLICATION

(11) **EP 2 248 513 A1**
(43) Date of publication of application: **10.11.2010**
(21) Application number: 09703681.8
(22) Date of filing: 23.01.2009
(51) Int. Cl.: A61K 8/46, A61K 8/25, A61K 8/34, A61K 8/81, A61K 8/92, A61Q 5/06

(54) **EMULSION-TYPE HAIR-DRESSING PREPARATION**

(30) Priority: 25.01.2008 JP 2008015580
(71) Applicant: Shiseido Company, Ltd., Chuo-ku Tokyo 104-8010 (JP)
(72) Inventor: TOYODA, Tomonori, Yokohama-shi Kanagawa 224-8558 (JP); SHIMIZU, Hideki, Yokohama-shi Kanagawa 224-8558 (JP); GOTO, Makiko, Yokohama-shi Kanagawa 224-8558 (JP); FUJIYAMA, Taizo, Yokohama-shi Kanagawa 224-8558 (JP)
(74) Representative: Gendron, Vincent Christian
(86) International application number: PCT/JP2009/051047
(87) International publication number: WO 2009/093677

(57) **Abstract**

Problem: To provide an oil-in-water emulsion-type hair dressing preparation which exhibits excellent spreadability, unclamminess and excellent hair-dressing properties and which does not generate a feeling of squeakiness.

Means for Resolution: An oil-in-water emulsion-type hair dressing preparation comprising (a) a specific long-chain acylsulfonic acid salt-type anionic surfactant (e.g., N-stearoyl-N-methyltaurine sodium) preferably in an amount of from 0.1 to 10% by mass, (b) a higher aliphatic alcohol preferably in an amount of from 0.1 to 20% by mass, (c) one or more selected from (c-1) wax in an amount of from 0.5 to 50% by mass, (c-2) hair-dressing resin in an amount of from 0.1 to 8% by mass, and (c-3) powdery ingredient (e.g., silicic anhydride) in an amount of from 0.1 to 8% by mass, and (d) water. This may further contain (e) a liquid oily matter.

## Description

### TECHNICAL FIELD

The present invention relates to an emulsion-type hair-dressing preparation. More precisely, the invention relates to an oil-in-water emulsion-type hair-dressing preparation which exhibits excellent spreadability, unclamminess and excellent hair-dressing properties and which does not generate a feeling of squeakiness.

### BACKGROUND ART

For hair cosmetic materials, heretofore used are oily matters such as wax, as well as hair-dressing resins (polymer compounds) and the like for the purpose of setting and fixing hair (for example, see Patent References 1 to 3). However, conventional hair cosmetics with wax or hair-dressing resin incorporated therein are problematic in that, when the amount of the wax or the resin to be therein is increased for the purpose of enhancing the fixing power of the cosmetic, then the hair-setting power thereof may increase, but with that, the feeling of roughness and clamminess increases, the smoothness reduces and the spreadability worsens.

Patent Reference 1: JP-A 10-45546
Patent Reference 2: JP-A 2002-12521
Patent Reference 3: JP-A 2004-67622

### DISCLOSURE OF THE INVENTION

### PROBLEMS THAT THE INVENTION IS TO SOLVE

The present invention has been made for the purpose of solving the above-mentioned problems, and its object is to provide an oil-in-water emulsion-type hair-dressing preparation which exhibits excellent spreadability, unclamminess and excellent hair-dressing properties and which does not generate a feeling of squeakiness.

### MEANS FOR SOLVING THE PROBLEMS

To attain the above-mentioned object, the invention provides an emulsion-type hair-dressing preparation comprising the following ingredients (a) to (d):

Component (a): Long-chain acylsulfonic acid salt type anionic surfactant represented by the following formula (I):

   R₁CO-a-(CH₂)ₙSO₃M₁ (I)

   [in formula (I), R₁CO- represents a saturated or unsaturated fatty acid residue (acyl group) having from 10 to 22 carbon atoms on average; a represents -O- or -NR₂- (where R₂ represents a hydrogen atom, or an alkyl group having from 1 to 3 carbon atoms); M₁ represents a hydrogen atom, an alkali metal, an alkaline earth metal, an ammonium or an organic amine; n indicates an integer of from 1 to 3];
Component (b): Higher aliphatic alcohol;
Component (c): one or more selected from (c-1) wax in an amount of from 0.5 to 50% by mass, (c-2) hair-dressing resin in an amount of from 0.1 to 8% by mass, and (c-3) powdery ingredient in an amount of from 0.1 to 8% by mass; and
Component (d): water.

In the above, preferably, the ratio of component (a)/component (b) is from 1/4 to 1/10 (by mol).

In the invention, the preparation may further contain (e) a liquid oily matter (oily matter that is liquid at 25°C).

### ADVANTAGE OF THE INVENTION

According to the invention, there is provided an oil-in-water emulsion-type hair-dressing preparation which exhibits excellent spreadability, unclamminess and excellent hair-dressing properties and which does not generate a feeling of squeakiness.

### BEST MODE FOR CARRYING OUT THE INVENTION

In the invention, a long-chain acylsulfonic acid salt type anionic surfactant represented by the following formula (I) is used as component (a).

R₁CO-A-(CH₂)ₙSO₃M₁ (I)

In the formula (I), R₁CO- represents a saturated or unsaturated fatty acid residue (acyl group) having from 10 to 22 carbon atoms on average. R₁CO includes C₁₁H₂₃CO, C₁₂H₂₅CO, C₁₃H₂₇CO, C₁₄H₂₉CO, C₁₅H₃₁CO, C₁₆H₃₃CO, C₁₇H₃₅CO, cocoyl group, and palmyl group. More preferably, R₁CO has from 12 to 22 carbon atoms on average from the viewpoint of the safety, etc.

a represents -O- or -NR- (where R represents a hydrogen atom, or an alkyl group having from 1 to 3 carbon atoms). These are an electron-donating group. a is preferably -O-, - NH- or -N(CH₃)-.

M₁ represents a hydrogen atom, an alkali metal, an alkaline earth metal, an ammonium or an organic amine. M₁ includes lithium, potassium, sodium, calcium, magnesium, ammonium, monoethanolamine, diethanolamine, triethanolamine, taurine sodium, and N-methyltaurine sodium.

n indicates an integer of from 1 to 3.

As component (a), the compound of formula (I) where a is -O-, or that is, a long-chain acylisethionic acid salt-type anionic surfactant includes cocoylisethionic acid salts, stearoylisethionic acid salts, laurylisethionic acid salts, and myristoylisethionic acid salts.

The compound of formula (I) where a is -NH-, or that is, a long-chain acyltaurine salt-type anionic surfactant includes N-lauroyltaurine salts, N-cocoyl-N-ethanoltaurine salts, N-myristoyltaurine salts, and N-stearoyltaurine salts.

The compound of formula (I) where a is -N(CH₃)-, or that is, a long-chain acylmethyltaurine salt-type anionic surfactant includes N-lauroyl-N-methyltaurine salts, N-palmitoyl-N-methyltaurine salts, N-stearoyl-N-methyltaurine salts, and N-cocoyl-N-methyltaurine salts.

In the invention, as component (a), preferred is a long-chain acylmethyltaurine salt-type anionic surfactant, and more preferred is an N-stearoyl-N-methyltaurine salt. One or more such components (a) may be in the preparation of the invention.

Not specifically defined, the higher aliphatic alcohol as component (b) in the invention may be any one usable in the field of cosmetics, medicines, and quasi drugs. For example, it includes saturated linear-chain monoalcohols, unsaturated monoalcohols, etc. The saturated linear-chain monoalcohols include dodecanol (= lauryl alcohol), tridecanol, tetradecanol (= myristyl alcohol), pentadecanol, hexadecanol (= cetyl alcohol), heptadecanol, octadecanol (=stearyl alcohol), nonadecanol, eicosanol (= arachyl alcohol), heneicosanol, docosanol (= behenyl alcohol), tricosanol, tetracosanol (= carnaubyl alcohol), pentacosanol, and hexacosanol (= ceryl alcohol). The unsaturated monoalcohols include eraidyl alcohol. In the invention, preferred are saturated linear-chain monoalcohols from the viewpoint of the stability, etc.

One or more such components (b) may be in the preparation of the invention; but from the viewpoint of the high-temperature stability, etc., the mean alkyl chain length as the arithmetic average is at least 18. The preferred uppermost limit of the mean alkyl chain length is not specifically defined, but preferably, however, the alkyl chain is up to around 22.

In the invention, a part or all of component (a) and component (b) form a gel in component (d) to be mentioned hereinunder. The amount of component (a) is preferably from 0.1 to 10% by mass of the total amount of the hair-dressing preparation more preferably from 0.5 to 1.5 % by mass, especially preferably from 1 to 3 % by mass. The amount of component (b) is preferably from 0.1 to 20% by mass of the total amount of the hair-dressing preparation, more preferably from 0.5 to 15% by mass, especially preferably from 3 to 20% by mass.

In the invention, the blend ratio of component (a) to component (b) is preferably component (a)/component (b) of being from 1/4 to 1/10 (by mol), for more effectively forming the gel. When the molar ratio oversteps the above range, then the gel could not be formed, and the total smoothness of the preparation base may decrease and the clamminess of the oily matter may increase.

In the invention, as component (c), used are one or more selected from (c-1) wax in an amount of from 0.5 to 50% by mass, (c-2) hair-dressing resin in an amount of from 0.1 to 8% by mass, and (c-3) powdery ingredient in an amount of from 0.1 to 8% by mass.

The wax for component (c-1) indicates a wax having a melting point not lower than 55°C and includes natural wax esters, such as candelilla wax, carnauba wax, bees wax, and lanoline; and synthetic was esters, such as paraffin wax, microcrystalline wax, polyethylene wax, polypropylene wax, and ceresin.

The amount of component (c-1) is preferably from 0.1 to 50% by mass of the total amount of the hair-dressing preparation, more preferably from 1 to 30% by mass, even more preferably from 7 to 20% by mass. When the amount is less than 0.1% by mass, then the preparation could not exhibit hair-dressing properties; but on the other hand, when the amount is more than 50% by mass, it is unfavorable since the preparation is clammy.

The hair-dressing resin for component (c-2) is not specifically defined. For example, there may be mentioned one or more selected from film-forming nonionic, anionic, ampholytic or cationic polymer compounds. Concretely, the following compounds may be mentioned. However, the ingredient is not limited to these examples.
(1) Vinylpyrrolidone-based polymer compounds, such as polyvinyl pyrrolidone, polyvinyl pyrrolidone/vinyl acetate copolymer, etc. (BASF's Luviskol K-90, VA73, etc.);
(2) Vinyl alcohol-based polymer compounds, such as polyvinyl alcohol, polyvinyl butyral, etc. (Nippon Gohsei's Gohsenol, Denki Kagaku Kogyo's Denka Poval, etc.);
(3) Acidic vinyl ether-based polymer compounds such as vinyl methyl ether/butyl maleate, etc. (Osaka Organic Chemical's Aniere BEM-42S, ISP's Gantrez ES-225, etc.);
(4) Acrylic acid-based polymer compounds, such as alkyl acrylate/alkyl methacrylate/diacetonacrylamide/methacrylic acid copolymer liquid, alkyl acrylate copolymer, acrylic acid/acrylic acid amide/ethyl acrylate copolymer, etc. (BASF's Luvimer 100P, Ultra Hold Strong; Mitsubishi Chemical's Dia Hold HR-200; Goo Chemical's Plus Size L-53P, etc.);
(5) Ampholytic acrylic acid-based polymer compounds, such as N-methacryloyloxyethyl-N,N-dimethylammonium-α-N-methylcarboxybetaine/alkyl methacrylate copolymer, acrylic acid octylamide/hydroxypropyl acrylate/butylaminoethyl methacrylate copolymer, etc. (Mitsubishi Chemical's Yukaformer AM-75, Nippon NSC's Amphomer 28-4910, etc.);
(6) Nitrogen-containing cationic polymer compounds, such as hydroxyethyl cellulose dimethyldiallylammonium chloride copolymer, hydroxyethyl cellulose hydroxypropyltrimethylammonium chloride, vinyl pyrrolidone/N,N'-dimethylaminoethylmethacrylic acid copolymer diethyl sulfate salt liquid, dimethyldiallylammonium chloride homopolymer, dimethyldiallylammonium chloride/acrylamide copolymer, etc. (Amerchol's Polymer JR-125, Lion's Leoguard GP, Nippon NSC's Celquat L-200, IPS's Gufquat 734, Osaka Organic Chemical's H. C. Polymer 1N, Nalco's Marquart 550, etc.).

The amount (actual content) of component (c-2) is from 0.1 to 8% by mass of the total amount of the hair-dressing preparation, preferably from 0.3 to 5% by mass, more preferably from 0.5 to 3% by mass. When the amount is less than 0.1% by mass, then the preparation could not exhibit hair-dressing properties; but on the other hand, when the amount is more than 8% by mass, it is unfavorable since the preparation is clammy.

Of the above-mentioned hair-dressing resin, when the anionic or ampholytic polymer is insoluble in water by itself, then if desired, a part or all of the functional groups therein may be neutralized with an inorganic or organic alkali agent to thereby make it soluble in water.

As the alkali agent for neutralization, the inorganic alkali agent includes an alkali metal hydroxide, such as sodium hydroxide, and potassium hydroxide. Also usable are volatile alkali agents, such as ammonia, and morpholine; alkanolamines, such as triethanolamine, diethanolamine, isopropanolamine, monoethanolamine, diisopropanolamine, 2-amino-2-methyl-1-propanol, and 2-amino-2-methyl-1,3-propanediol; amino acids, such as L-arginine, and lysine.

Not specifically defined, the powdery ingredient for component (c-3) may be any one generally used in cosmetics, and includes inorganic powders, such as talc, mica, kaolin, sericite, muscovite, phlogopite, synthetic mica, lepidolite, biotite, lepidolite, vermiculite, magnesium carbonate, calcium carbonate, aluminium silicate, barium silicate, calcium silicate, magnesium silicate, strontium silicate, metal tungstate, magnesium, spherical silica, silicic anhydride [e.g., "Aerosil #200" (by Nippon Aerosil), etc.], silylated silicic anhydride [e.g., Aerosil #972" (by Nippon Aerosil), etc.], zeolite, barium sulfate, fired calcium sulfate (burnt plaster), calcium phosphate, fluoroapatite, hydroxyapatite, ceramic powder, metal soap (zinc myristate, calcium palmitate, aluminium stearate, etc.), and boron nitride; spherical organic powders, such as polyamide spherical resin powder (nylon spherical powder), spherical polyethylene, crosslinked polymethyl (meth)acrylate spherical resin powder, spherical polyester, crosslinked polystyrene spherical resin powder, styrene/acrylic acid copolymer spherical resin powder, benzoguanamine spherical resin powder, polytetrafluoroethylene spherical powder, and spherical cellulose; inorganic white pigments, such as titanium dioxide, and zinc oxide; inorganic red pigments, such as iron oxide (Bengal red), and iron titanate; inorganic brown pigments such as γ-iron oxide; inorganic yellow pigments, such as yellow iron oxide, and Chinese yellow; inorganic black pigments, such as black iron oxide, carbon black, and low-order titanium oxide; inorganic violet pigments, such as mango violet, and cobalt violet; inorganic green pigments, such as chromium oxide, chromium hydroxide, and cobalt titanate; inorganic blue pigments, such as ultramarine, and Prussian blue; pearly pigments, such as titanium oxide-coated mica, titanium oxide-coated bismuth oxychloride, titanium oxide-coated talc, colored titanium oxide-coated mica, bismuth oxychloride, and argentine; metal powder pigments, such as aluminium powder, and copper powder; colorants (organic pigments) of red, yellow, orange, yellow, green, blue, etc., or their laked colorants with zirconium, barium, aluminium; natural dyes, such as chlorophyll, and β-carotene. Above all, preferred are kaolin, silicic anhydride ("Aerosil #200"), silylated silicic anhydride ("Aerosil #972"), etc., from the viewpoint of the handlability, etc.

The amount of component (c-3) is preferably from 0.1 to 8% by mass of the total amount of the hair-dressing preparation, more preferably from 0.3 to 5% by mass, even more preferably from 0.5 to 3% by mass. When the amount is less than 0.1% by mass, then the preparation could not exhibit hair-dressing properties; but on the other hand, when the amount is more than 3% by mass, it is unfavorable since the preparation generates a feeling of squeakiness.

Of component (c), the wax of component (c-1) is effective for arrangement in hair-styling, the hair-dressing resin of component (c-2) is effective for keeping the arranged hairstyle and for straight rising thereof, and the powder of component (c-3) is effective for partial exhibition of lightness and soft feel of hairstyle. Accordingly, the emulsion-type hair-dressing preparation of the invention may contain any of these components (c-1) to (c-3) in accordance with the object and the use thereof and with the intended effect thereof.

The hair-dressing preparation of the invention is an oil-in-water emulsion-type hair-dressing preparation, and water of component (d) therein is the main component of the outer phase (aqueous phase). In the invention, preferably, the outer phase (aqueous phase) accounts for from 40 to 80% by mass, and the inner phase (oily phase) accounts for from 5 to 50% by mass or so.

In the invention, the preparation may further contain (e) a liquid oily matter (oily matter that is liquid at 25°C) as the oily phase ingredient therein. Not specifically defined, the liquid oily matter may be any one generally used in hair cosmetics, and includes hydrocarbon oils, such as heavy isoparaffin (= hydrogenated polyisobutene), squalane, and liquid paraffin; esters, such as cetyl-2-ethylhexanoate, 2-ethylhexyl palmitate, 2-octyldodecyl myristate, neopentyl glycol-2-ethylhexanoate, isopropyl myristate, myristyl myristate, and pentaerythrityl tetra-2-ethylhexanoate; oils and fats, such as olive oil, avocado oil, jojoba oil, sunflower oil, safflower oil, camellia oil, macadamia nut oil, mink oil, liquid lanolin, lanolin acetate, and castor oil; silicone oils, such as dimethylpolysiloxane, methylphenylpolysiloxane, high-polymerization gummy dimethylpolysiloxane, polyether-modified silicone, and amino-modified silicone; fluorine-containing oily matters, such as fluorine-modified dimethylpolysiloxane, fluorine-modified methylphenylpolysiloxane, and perfluoropolyether, perfluorocarbon; One or more such components (e) may be in the preparation of the invention.

In cases where component (e) is incorporated in the preparation, its amount is preferably from 1 to 30% by mass of the total amount of the hair-dressing preparation, more preferably from 2 to 25% by mass, even more preferably from 5 to 20% by mass, from the viewpoint that the preparation exhibits hair-dressing properties and is not clammy.

The hair-dressing preparation of the invention may further contain any other ingredients that may be incorporated in ordinary cosmetics and quasi drugs, within a range not detracting from the effect of the invention. The ingredients include, for example, surfactant (except the above-mentioned ingredient (a)), moisturizing agent, volatile oil (oil volatile at 25°C), dispersant, preservative, fragrance, medicament, and UV absorbent.

The surfactant includes anionic surfactants, such as sodium salts, potassium salts, triethanolamine salts, alkyl sulfate ester salts, polyoxyethylene alkyl ether sulfate salts, alkyl ether phosphate salts and the like of higher fatty acids such as 2-ethylhexanoic acid, caproic acid, caprylic acid, lauric acid, myristic acid, palmitic acid, 2-palmitoleic acid, stearic acid, isostearic acid, oleic acid, linolic acid, linolenic acid, ricinoleic acid, arachic acid, behenic acid, petroselinic acid, elaidic acid, linoelaidic acid, arachidonic acid, 12-hydroxystearic acid, etc.; cationic surfactants such as alkyltrimethylammonium chloride, dialkyldimethylammonium chloride, and benzalkonium chloride; ampholytic surfactants, such as alkyldimethylaminoacetic acid betaine, alkylamidodimethylaminoacetic acid betaine, and alkylcarboxyhydroxyimidazolinium betaine; nonionic surfactants, such as polyoxyethylene-added higher fatty acid esters, higher aliphatic alcohol esters, polyalcohol esters, and ethylene oxide/propylene oxide block copolymers; polysurfactants, etc. In cases where the other surfactant than component (a) is incorporated in the preparation of the invention, its amount is preferably small for the purpose of keeping the good handlability of the preparation such as the unclamminess and the spreadability thereof.

The moisturizing agent includes polyalcohols, such as dynamite glycerin, 1,3-butylene glycol, dipropylene glycol, and propylene glycol; water-soluble polymers, such as hyaluronic acid, and chondroitin sulfate. Its amount to be in the preparation is preferably from 0.1 to 65% by mass.

As the volatile oil, preferably used herein are a low-boiling-point (having a boiling point of not higher than 260°C under normal pressure) isoparaffin hydrocarbon oils, low-boiling-point silicone oils, etc. The low-boiling-point isoparaffin hydrocarbon oils (light isoparaffins) concretely include commercial products of Isopar A, C, E, G, H, K, L and M (all by Exxon), Shellsol 71 (by Shell), Soltrol 100, 130 and 220 (all by Phillips). The low-boiling-point silicone oils include hexamethylcyclotrisiloxane, octamethyltetracyclosiloxane, decamethylcyclopentasiloxane, dodecamethylcyclohexasiloxane, and tetradecamethylcycloheptasiloxane.

The hair-dressing preparation of the invention can be produced by mixing the constitutive ingredients and emulsifying them according to a known method, for example, according to a phase transfer emulsification method using a homomixer. However, the invention is not limited to the production method. The mixing and the emulsification may be attained separately or simultaneously.

The oil-in-water emulsion-type hair-dressing preparation of the invention is applicable to various preparation forms of wax, cream, gel, emulsion or the like, and is favorably used as hair wax, hair milk, hair cream, etc.

### EXAMPLES

The invention is described in detail with reference to the following Examples, by which, however, the invention is not limited at all. Unless otherwise specifically indicated, the amount of the constitutive ingredient is expressed by % by mass of the system in which the ingredient is incorporated.

### (Example 1, Comparative Example 1)

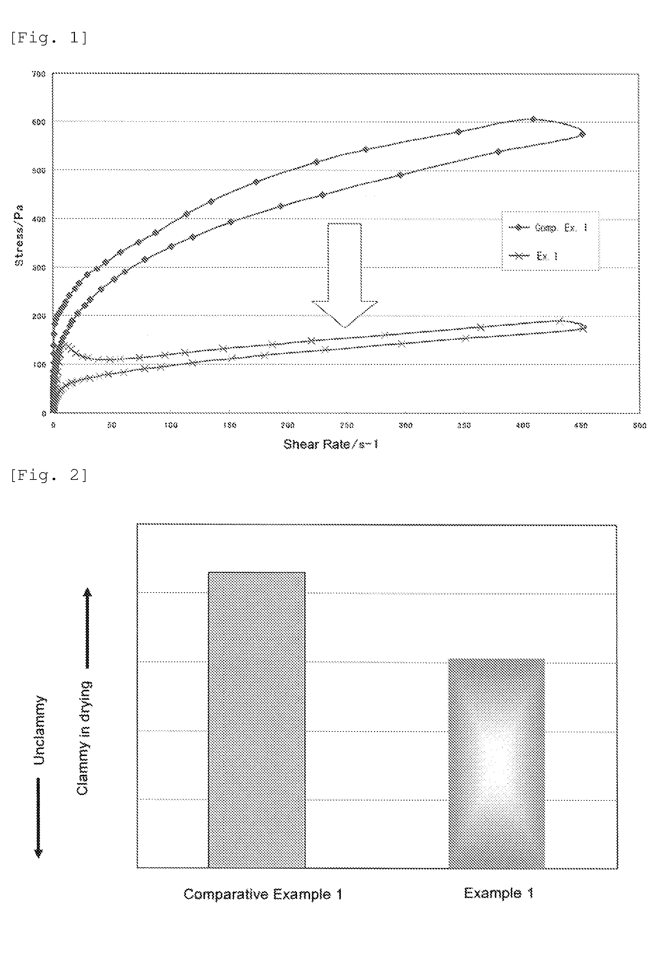

Oil-in-water emulsion-type hair-dressing preparations of Example 1 and Comparative Example 1 each having the composition shown below were produced, and these were tested for the spreadability (dynamic viscosity behavior) and the clamminess in drying after application (rolling friction) according to the test method and the evaluation standard mentioned below. The results are shown in Fig. 1 and Fig. 2. The preparation of Comparative Example 1 does not contain component (a).

**Example 1:**

| (Constitutive Ingredients) | (% by mass) |
|---|---|
| (1) Candelilla wax | 2.0 |
| (2) Microcrystalline wax | 12.0 |
| (3) Liquid paraffin | 3.5 |
| (4) Hydrogenated polyisobutene | 3.5 |
| (5) Pentaerythrityl tetra-2-ethylhexanoate | 3.0 |
| (6) PEG-60 glyceryl isostearate | 1.0 |
| (7) Glyceryl stearate | 1.0 |
| (8) Behenyl alcohol | 3.3 |
| (9) Stearyl alcohol | 0.9 |
| (10) Tocopherol | 0.5 |
| (11) Fragrance | 0.1 |
| (12) Ion-exchanged water | balance |
| (13) Propylene glycol | 8.0 |
| (14) N-stearoyl-N-methyltaurine sodium | 1.2 |
| (15) Silicic anhydride | 2.5 |
| (16) Triethanolamine | 0.4 |
| (17) Polyvinyl pyrrolidone/vinyl acetate copolymer | 1.8 |

### (Production Method)

(1) to (11) were stirred and dissolved at 85°C (oily phase part). On the other hand, (12) to (14) were stirred and dissolved at 75°C (aqueous phase part). The oily phase part was added to the aqueous phase part and emulsified, and then (15) was added thereto. Next, (16) was added and neutralized, and then (17) was added, degassed, and cooled.

**Comparative Example 1:**

| (Constitutive Ingredients) | (% by mass) |
|---|---|
| (1) Ion-exchanged water | balance |
| (2) Propylene glycol | 10.0 |
| (3) Carboxyvinyl polymer | 0.1 |
| (4) Sodium polyacrylate | 0.05 |
| (5) EDTA-2Na.2H₂O | 0.02 |
| (6) POE hardened castor oil (60 E.O.) | 3.0 |
| (7) Glyceryl stearate | 2.0 |
| (8) POE oleyl ether phosphoric acid (10 E.O.) | 2.0 |
| (9) Stearic acid (plant oil-type) | 4.5 |
| (10) Isostearic acid | 1.0 |
| (11) Liquid paraffin | 3.0 |
| (12) Synthetic isoparaffin | 6.0 |
| (13) Pentaerythrityl tetra-2-ehtylhexanoate | 5.0 |
| (14) Methylpolysiloxane | 0.5 |
| (15) Microcrystalline wax | 7.0 |
| (16) Carnauba wax | 3.0 |
| (17) Candelilla wax | 3.0 |
| (18) Stearyl alcohol | 1.0 |
| (19) Tocopherol | 0.05 |
| (20) Phenoxyethanol | 0.5 |
| (21) Fragrance | 0.1 |
| (22) Triethanolamine | 1.8 |
| (23) Butylaminoethyl methacrylate | 1.2 |
| (24) Butylaminoethyl methacrylate copolymer | 0.3 |

### (Production Method)

(1) to (5) were stirred and dissolved at 70 to 80°C (aqueous phase part). On the other hand, (6) to (21) were stirred and dissolved at 75°C (oily phase part). The oily phase part was added to the aqueous phase part and emulsified, and then (22) was added thereto and neutralized, and then (23) and (24) were added, degassed, and cooled.

### [Spreadability (dynamic viscosity behavior)]

The sample of Example 1 and Comparative Example 1 was given stress, whereupon the relationship between the stress and the shear rate (dynamic viscosity) was monitored with a cone-plate rheometer.

### [Clamminess in drying after application (rolling friction)]

The sample of Example 1 and Comparative Example 1 was put on a horizontally movable plate, and the rolling power acting on a probe was measured to determine the friction between the sample and the probe.

As obvious from the results in Fig. 1, the stress of the sample is lower when pressure is applied thereto in Example 1, as compared with that in Comparative Example 1; and this confirms good spreadability of the preparation (sample) in Example 1.

As obvious from the results in Fig. 2, the rolling friction is lower in Example 1 than in Comparative Example 1; and this confirms the unclamminess of the preparation of Example 1 in drying. In Fig 2, the vertical axis indicates the degree of rolling friction. When the graph is higher, the degree of rolling friction is higher.

### (Comparative Example 2, Examples 2 to 6)

Hair-dressing preparations each having the composition shown in Table 1 were produced according to an ordinary method, and actually tried and tested for the hair-dressing properties, the clamminess, the spreadability and the feeling of squeakiness, under the evaluation standards shown below. The results are shown in Table 1.

### <Hair-Dressing Properties>

The samples were actually tried by expert panelists (20 persons), and tested for natural hair-dressing properties thereof to give natural hair streamline (especially in point of the arranging effect in hairstyling) and for the sustainability thereof. The evaluation method is as follows: Each panelist gave points under the following standards, and the points were summed up to give the scores of the tested samples.

### (Evaluation Point)

Point 5: Very good
Point 4: Good
Point 3: Average
Point 2: Bad
Point 1: Very bad

### (Evaluation Standard)

⊙: The total score is 90 points or more.
○: The total score is from 60 points to less than 90 points.
△: The total score is from 30 points to less than 60 points.
×: The total score is less than 30 points.

### <Clamminess>

The samples were actually tried by expert panelists (20 persons) and evaluated for unclamminess to fingers. The evaluation method is as follows: Each panelist gave points under the following standards, and the points were summed up to give the scores of the tested samples.

### (Evaluation Point)

Point 5: Very good
Point 4: Good
Point 3: Average
Point 2: Bad
Point 1: Very bad

### (Evaluation Standard)

⊙: The total score is 90 points or more.
○: The total score is from 60 points to less than 90 points.
△: The total score is from 30 points to less than 60 points.
×: The total score is less than 30 points.

### <Spreadability>

The samples were actually tried by expert panelists (20 persons) and evaluated for light spreadability and smooth finger combing. The evaluation method is as follows: Each panelist gave points under the following standards, and the points were summed up to give the scores of the tested samples.

### (Evaluation Point)

Point 5: Very good
Point 4: Good
Point 3: Average
Point 2: Bad
Point 1: Very bad

### (Evaluation Standard)

⊙: The total score is 90 points or more.
○: The total score is from 60 points to less than 90 points.
△: The total score is from 30 points to less than 60 points.
×: The total score is less than 30 points.

### <Feeling of Squeakiness>

The samples were actually tried by expert panelists (20 persons) and evaluated for smooth finger combing with neither difficulty in finger combing nor entangling of hair around fingers. The evaluation method is as follows: Each panelist gave points under the following standards, and the points were summed up to give the scores of the tested samples.

### (Evaluation Point)

Point 5: Very good
Point 4: Good
Point 3: Average
Point 2: Bad
Point 1: Very bad

### (Evaluation Standard)

⊙: The total score is 90 points or more.
○: The total score is from 60 points to less than 90 points.
△: The total score is from 30 points to less than 60 points.
×: The total score is less than 30 points.

**[Table 1]**

| | Comp. Ex. 2 | Ex. 2 | Ex. 3 | Ex. 4 | Ex. 5 | Ex. 6 |
|---|---|---|---|---|---|---|
| Ion-exchanged water [component (d)] | balance | balance | balance | balance | balance | balance |
| Propylene glycol | 8 | 8 | 8 | 8 | 8 | 8 |
| PEG-60 glyceryl isostearate | 1 | 1 | 1 | 1 | 1 | 1 |
| Glyceryl stearate | 1 | 1 | 1 | 1 | 1 | 1 |
| Liquid paraffin [component (e)] | 3.5 | 3.5 | 3.5 | 3.5 | 3.5 | 3.5 |
| Hydrogenated polyisobutene [component (e)] | 3.5 | 3.5 | 3.5 | 3.5 | 3.5 | 3.5 |
| Pentaerythrityl tetra-2-ethylhexanoate [component (e)] | 3 | 3 | 3 | 3 | 3 | 3 |
| Phenoxyethanol | 0.5 | 0.5 | 0.5 | 0.5 | 0.5 | 0.5 |
| Tocopherol | 0.05 | 0.05 | 0.05 | 0.05 | 0.05 | 0.05 |
| Fragrance | 0.1 | 0.1 | 0.1 | 0.1 | 0.1 | 0.1 |
| Stearoylmethyltaurine sodium [component (a)] | 1 | 1 | 1 | 1 | 1 | 1 |
| Behenyl alcohol [component (b)] | 3 | 3 | 3 | 3 | 3 | 3 |
| Stearyl alcohol [component (b)] | 1 | 1 | 1 | 1 | 1 | 1 |
| Candelilla wax [component (c-1)] | - | - | 2 | 9 | 12 | 15 |
| Microcrystalline wax [component (c-1)] | 0.1 | 1 | 7 | 2 | 2 | 15 |
| Component (b)/Component (a) (ratio by mol) | 5 | 5 | 5 | 5 | 5 | 5 |
| Hair-dressing properties | × | △ | ○ | ○ | ⊙ | ⊙ |
| Clamminess | ○ | ○ | ○ | ○ | ○ | △ |
| Spreadability | ○ | ○ | ○ | ○ | ○ | △ |
| Feeling of squeakiness | ○ | ○ | ○ | ○ | ○ | ○ |

### (Comparative Examples 3 to 4, Example 7 to 10)

Hair-dressing preparations each having the composition shown in Table 2 were produced according to an ordinary method, and tried and tested under the evaluation standards mentioned above, for the hair-dressing properties (especially for the effect of keeping the arranged hairstyle and systaining the Straight rising harstyle) clamminess, spreadability and feeling of squeakiness. The results are shown in Table 2.

**[Table 2]**

| | Comp. Ex. 3 | Ex. 7 | Ex. 8 | Ex. 9 | Ex. 10 | Comp. Ex. 4 |
|---|---|---|---|---|---|---|
| Ion-exchanged water [component (d)] | balance | balance | balance | balance | balance | balance |
| Propylene glycol | 8 | 8 | 8 | 8 | 8 | 8 |
| PEG-60 glyceryl isostearate | 1 | 1 | 1 | 1 | 1 | 1 |
| Glyceryl stearate | 1 | 1 | 1 | 1 | 1 | 1 |
| Liquid paraffin [component (e)] | 3.5 | 3.5 | 3.5 | 3.5 | 3.5 | 3.5 |
| Hydrogenated polyisobutene [component (e)] | 3.5 | 3.5 | 3.5 | 3.5 | 3.5 | 3.5 |
| Pentaerythrityl tetra-2-ethylhexanoate [component (e)] | 3 | 3 | 3 | 3 | 3 | 3 |
| Phenoxyethanol | 0.5 | 0.5 | 0.5 | 0.5 | 0.5 | 0.5 |
| Tocopherol | 0.05 | 0.05 | 0.05 | 0.05 | 0.05 | 0.05 |
| Fragrance | 0.1 | 0.1 | 0.1 | 0.1 | 0.1 | 0.1 |
| Stearoylmethyltaurine sodium [component (a)] | 1 | 1 | 1 | 1 | 1 | 1 |
| Behenyl alcohol [component (b)] | 3 | 3 | 3 | 3 | 3 | 3 |
| Stearyl alcohol [component (b)] | 1 | 1 | 1 | 1 | 1 | 1 |
| Polyvinyl pyrrolidone/vinyl acetate copolymer [component (c-2)] | - | 0.1 | 0.5 | 1 | 5 | 10 |
| Component (b)/Component (a) (ratio by mol) | 5 | 5 | 5 | 5 | 5 | 5 |
| Hair-dressing properties | × | △ | ○ | ⊙ | ⊙ | ⊙ |
| Clamminess | ○ | ○ | ○ | ○ | △ | × |
| Spreadability | ○ | ○ | ○ | ○ | ○ | △ |
| Feeling of squeakiness | ○ | ○ | ○ | ○ | △ | △ |

### (Comparative Examples 5 to 6, Examples 11 to 14)

Hair-dressing preparations each having the composition shown in Table 3 were produced according to an ordinary method, and tried and tested under the evaluation standards mentioned above for the hair-dressing properties (especially for lightness and soft feel of hairstyle), clamminess, spreadability and feeling of squeakiness. The results are shown in Table 3.

**[Table 3]**

| | Comp. Ex. 5 | Ex. 11 | Ex. 12 | Ex. 13 | Ex. 14 | Comp. Ex. 6 |
|---|---|---|---|---|---|---|
| Ion-exchanged water [component (d)] | balance | balance | balance | balance | balance | balance |
| Propylene glycol | 8 | 8 | 8 | 8 | 8 | 8 |
| PEG-60 glyceryl isostearate | 1 | 1 | 1 | 1 | 1 | 1 |
| Glyceryl stearate | 1 | 1 | 1 | 1 | 1 | 1 |
| Liquid paraffin [component (e)] | 3.5 | 3.5 | 3.5 | 3.5 | 3.5 | 3.5 |
| Hydrogenated polyisobutene [component (e)] | 3.5 | 3.5 | 3.5 | 3.5 | 3.5 | 3.5 |
| Pentaerythrityl tetra-2-ethylhexanoate [component (e)] | 3 | 3 | 3 | 3 | 3 | 3 |
| Phenoxyethanol | 0.5 | 0.5 | 0.5 | 0.5 | 0.5 | 0.5 |
| Tocopherol | 0.05 | 0.05 | 0.05 | 0.05 | 0.05 | 0.05 |
| Fragrance | 0.1 | 0.1 | 0.1 | 0.1 | 0.1 | 0.1 |
| Stearoylmethyltaurine sodium [component (a)] | 1 | 1 | 1 | 1 | 1 | 1 |
| Behenyl alcohol [component (b)] | 3 | 3 | 3 | 3 | 3 | 3 |
| Stearyl alcohol [component (b)] | 1 | 1 | 1 | 1 | 1 | 1 |
| Silicic anhydride [component (c-3)] | - | 0.1 | 0.5 | 1 | 5 | 10 |
| Component (b)/Component (a) (ratio by mol) | 5 | 5 | 5 | 5 | 5 | 5 |
| Hair-dressing properties | × | △ | ○ | ⊙ | ⊙ | ⊙ |
| Clamminess | ○ | ○ | ○ | ○ | ○ | ○ |
| Spreadability | ○ | ○ | ○ | ○ | ○ | △ |
| Feeling of squeakiness | ○ | ○ | ○ | ○ | △ | × |

### (Comparative Examples 7 to 8, Examples 15 to 24)

Hair-dressing preparations each having the composition shown in Table 4 were produced according to an ordinary method, and tried and tested under the evaluation standards mentioned above for the hair-dressing properties, clamminess, spreadability ity and feeling of squeakiness. The results are shown in Table 4.

**[Table 4]**

| | Comp. Ex. 7 | Ex. 15 | Ex. 16 | Ex. 17 | Ex. 18 | Comp. Ex. 8 | Ex. 19 | Ex. 20 | Ex. 21 | Ex. 22 | Ex. 23 | Ex. 24 |
|---|---|---|---|---|---|---|---|---|---|---|---|---|
| Ion-exchanged water [component (d)] | bal. | bal. | bal. | bal. | bal. | bal. | bal. | bal. | bal. | bal. | bal. | bal. |
| Propylene glycol | 8 | 8 | 8 | 8 | 8 | 8 | 8 | 8 | 8 | 8 | 8 | 8 |
| PEG-60 glyceryl isostearate | 1 | 1 | 1 | 1 | 1 | 1 | 1 | 1 | 1 | 1 | 1 | 1 |
| Glyceryl stearate | 1 | 1 | 1 | 1 | 1 | 1 | 1 | 1 | 1 | 1 | 1 | 1 |
| Liquid paraffin [component (e)] | 3.5 | 3.5 | 3.5 | 3.5 | 3.5 | 3.5 | 3.5 | 3.5 | 3.5 | 3.5 | 3.5 | 3.5 |
| Hydrogenated polyisobutene [component (e)] | 3.5 | 3.5 | 3.5 | 3.5 | 3.5 | 3.5 | 3.5 | 3.5 | 3.5 | 3.5 | 3.5 | 3.5 |
| Pentaerythrityl tetra-2-ethylhexanoate [component (e)] | 3 | 3 | 3 | 3 | 3 | 3 | 3 | 3 | 3 | 3 | 3 | 3 |
| Phenoxyethanol | 0.5 | 0.5 | 0.5 | 0.5 | 0.5 | 0.5 | 0.5 | 0.5 | 0.5 | 0.5 | 0.5 | 0.5 |
| Tocopherol | 0.05 | 0.05 | 0.05 | 0.05 | 0.05 | 0.05 | 0.05 | 0.05 | 0.05 | 0.05 | 0.05 | 0.05 |
| Fragrance | 0.1 | 0.1 | 0.1 | 0.1 | 0.1 | 0.1 | 0.1 | 0.1 | 0.1 | 0.1 | 0.1 | 0.1 |
| Stearoylmethyltaurine sodium [component (a)] | 1 | 1 | 1 | 1 | 1 | 1 | 1 | 1 | 1 | 1 | 1 | 1 |
| Behenyl alcohol [component (b)] | 3 | 3 | 3 | 3 | 3 | 3 | 3 | 3 | 3 | 3 | 3 | 3 |
| Stearyl alcohol [component (b)] | 1 | 1 | 1 | 1 | 1 | 1 | 1 | 1 | 1 | 1 | 1 | 1 |
| Candelilla wax [component (c-1)] | - | 12 | 12 | - | - | - | 1 | 12 | 15 | 12 | 12 | 12 |
| Polyvinyl pyrrolidone/vinyl acetate copolymer [component (c-2)] | - | 0.1 | 5 | 5 | 5 | 5 | 5 | 5 | 5 | 5 | 5 | 5 |
| Silicic anhydride [component (c-3)] | - | - | - | 0.1 | 1 | 10 | - | - | - | 0.1 | 1 | 5 |
| Component (b)/Component (a) (ratio by mol) | 5 | 5 | 5 | 5 | 5 | 5 | 5 | 5 | 5 | 5 | 5 | 5 |
| Hair-dressing properties | × | ○ | ⊙ | △ | ○ | ⊙ | ○ | ⊙ | ⊙ | ⊙ | ⊙ | ⊙ |
| Clamminess | ○ | ○ | ○ | ○ | ○ | △ | ○ | ○ | △ | ○ | ○ | △ |
| Spreadability | ○ | ○ | ○ | ○ | △ | ○ | ○ | ○ | △ | ○ | △ | △ |
| Feeling of squeakiness | ○ | ○ | ○ | ○ | ○ | × | ○ | ○ | △ | ○ | ○ | △ |
| Comprehensive Evaluation | × | ○ | ⊙ | ○ | ○ | △ | ○ | ⊙ | △ | ⊙ | ○ | △ |

### BRIEF DESCRIPTION OF THE DRAWINGS

[Fig. 1] It is a drawing showing the relationship between the stress and the shear rate in Example 1 and Comparative Example 1.
[Fig. 2] It is a graph showing the degree of rolling friction in Example 1 and Comparative Example 1.

### INDUSTRIAL APPLICABILITY

According to the invention, there is provided an oil-in-water emulsion-type hair-dressing preparation which exhibits excellent spreadability, unclamminess an excellent hair-dressing properties and which does not generate a feeling of squeakiness.

## Claims

1. An emulsion-type hair-dressing preparation comprising the following components (a) to (d):
Component (a): Long-chain acylsulfonic acid salt type anionic surfactant represented by the following formula (I):
R₁CO-a-(CH₂)ₙSO₃M₁ (I)
[in formula (I), R₁CO- represents a saturated or unsaturated fatty acid residue (acyl group) having from 10 to 22 carbon atoms on average; a represents -O- or -NR₂- (where R₂ represents a hydrogen atom, or an alkyl group having from 1 to 3 carbon atoms); M₁ represents a hydrogen atom, an alkali metal, an alkaline earth metal, an ammonium or an organic amine; n indicates an integer of from 1 to 3];
Component (b): Higher aliphatic alcohol;
Component (c): one or more selected from (c-1) wax in an amount of from 0.5 to 50% by mass, (c-2) hair-dressing resin in an amount of from 0.1 to 8% by mass, and (c-3) powdery ingredient in an amount of from 0.1 to 8% by mass; and
Component (d): water.

2. The emulsion-type hair-dressing preparation as claimed in claim 1, wherein a in the formula (I) is -NH₃-.

3. The emulsion-type hair-dressing preparation as claimed in claim 1, which contains component (a) in an amount of from 0.1 to 10% by mass and component (b) in an amount of from 0.1 to 20% by mass.

4. The emulsion-type hair-dressing preparation as claimed in any one of claims 1 to 3, wherein the ratio of component (a)/component (b) is from 1/4 to 1/10 (by mol).

5. The emulsion-type hair-dressing preparation as claimed in claim 1, wherein component (c-3) is one or more selected from kaolin, silicic anhydride and silylated silicic anhydride.

6. The emulsion-type hair-dressing preparation as claimed in claim 1, which further contains (e) a liquid oily matter (oily matter liquid at 25°C).
